# EUROPEAN PATENT APPLICATION

(11) **EP 1 243 287 A1**
(43) Date of publication of application: **25.09.2002**
(21) Application number: 00985272.4
(22) Date of filing: 21.12.2000
(51) Int. Cl.: A61N 5/06

(54) **THERMOSTIMULATION APPARATUS FOR THERAPEUTIC TREATMENTS**

(30) Priority: 28.12.1999 ES 9903257 U
(71) Applicant: Villalon Castilla, Antonia, 17600 Figueres (Gerona) (ES)
(72) Inventor: Villalon Castilla, Antonia, 17600 Figueres (Gerona) (ES)
(74) Representative: Pons Arino, Angel
(86) International application number: ES0000481
(87) International publication number: WO01047601

(57) **Abstract**

Thermostimulating device for therapeutic treatments, made up of an equipment which includes an electronic unit (1), of digital or analogue type, in the functions of control module for the generation process of infrared emission that is projected by means of an issuing element for the application on treatment areas or points being determinable the process of infrared emission selectively in a continuous way of through impulses, for its application.

## Description

For diversity of therapeutic treatments, the use of acupuncture and lasertherapy techniques is known by means of stimulating some concrete points of the body.

According to the present invention, a device for similar application is proposed, but based on a thermostimulating practice by infrared radiation, with which an effective technique is reached without the risk of injuries or burns for the patient.

The mentioned technique can be applied for numerous treatments, having verified its use for example, in the de-acclimatation of tobacco-addiction, stress, obesity, insomnia, migraines, impotence, pain, drug-addiction and alcoholism, without discarding the application for other treatments with equally positive results.

The aforementioned apparatus, object of the invention, consists in a set made up of an electronic unit, such as a module for the control of the infrared emission process to be applied, and an issuing element, such as a terminal for the application of the infrared projection on the treatment points.

The electronic unit is of a digital or analogue type, with an operation control through a microprocessor, having the command and control elements which allow a simple and practical use in some adequate conditions and in a precise way for the functions to be developed, with the possibility to establish selectively a emission process which is continuous or through impulses for the infrared projection to be applied.

The infrared projection issuing element can be connected by means of a plug to the electronic control unit, incorporating in turn an activation control for the operation.

This away a simple device is obtained, which is easy to handle, by means of which a termostimulating action Can be carried out with therapeutic application for the treatment of affections which can be controlled by means of incidence on certain points or areas of the body.

Thus, the mentioned device, object of the invention has some very advantageous features, acquiring own life and preferable character for the therapeutic treatment function for which it is destined.

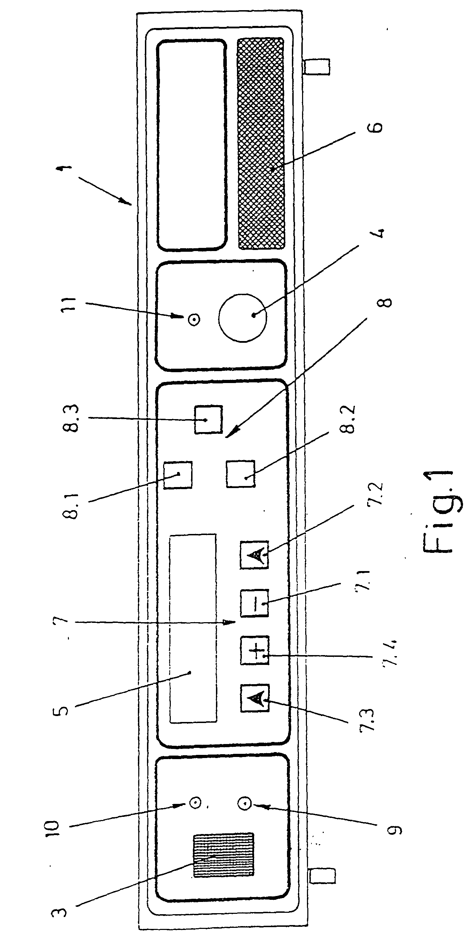

Figure 1 shows a front view of the electronic unit of the preconized device.

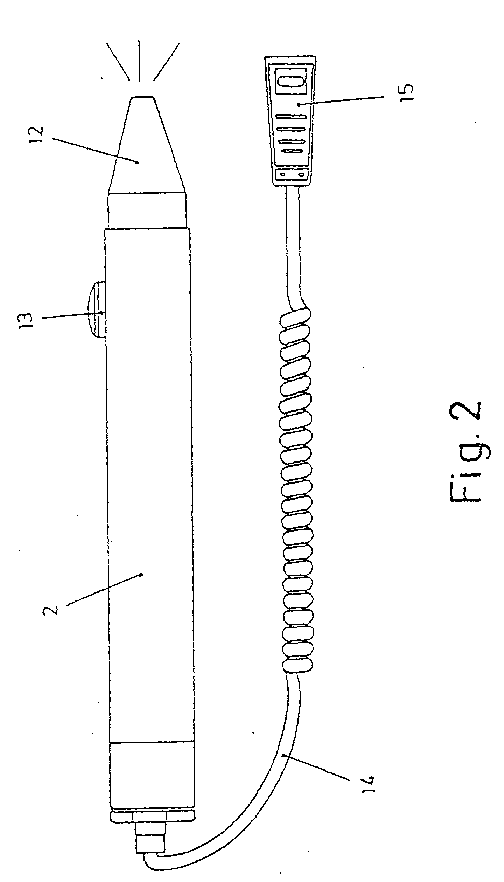

Figure 2 is a side view of the issuing element of the infrared projection to be applied.

The object of the invention refers to an apparatus destined to the therapeutic application of thermostimulations to be used for the treatment of, for example tobacco-addiction, stress, obesity, insomnia, migraines, impotence, pain, drug-addiction, alcoholism, etc...

The device consists in a set made up of an electronic unit (1), of a digital or analogue type, which is foreseen as a control module for the infrared emission process, which can be projected by means of an issuing element (2), for the application on the points or areas to be treated.

The electronic unit (1) can be connected to a 220 V mains network; it incorporates the needed equipment to transform and rectify the entrance current, so that the component elements of the set remain fed with a low tension continuous current. Of course the electric feeding could also be carried out by means of batteries, without therefore altering the concept of the device.

According to a particular, non limitative realisation, referring to its distribution, the mentioned electronic unit (1) includes an on - off push-button (3) to connect and disconnect the element, a connector (4) to connect the issuing element (2), a Signalling screen (5), an acoustic beeper (6), a set (7) of function keys, a set (8) of programming keys and the repective service indicating devices (9), to start up (10) and to control the impulses (11).

The issuer (2) itself includes a pointing devise (12) to project the infrared emission, an activating push-button (13), a cable (14) to connect the unit (1) and a terminal (15) to establish the connection respect to the connecting device (4) of the mentioned unit (1).

With all this, an issuer (2) to use the device is connected to the electronic unit (1) by means of the connection of the terminal (15) in the connecting device (4); establishing for its operation the corresponding power supply.

The star up is activated with the push button (3), which is foreseen of own signalling lighting and moreover related to the acoustic beeper (6) to indicate, by means of some beeps, the activation of the equipment.

When the equipment is activated, apart from the push-button (3) the service indicator (9) and the screen (5) remain lighted; the last treatment carried out by the equipment appears on the latter in principle, showing afterwards a self-checking signal, so that if everything is correct it remains reflected on the mentioned screen (5) and in the case of some failure, in this case this remains indicated on the same.

If the set is prepared with user's password, the mentioned password should be introduced for the process to be operative. On screen (5) in a case appears the password request, when this is necessary, also reflecting the operations which have to follow next.

To start the application treatment the push-button (13) of the issuer (2) should be pushed, with which on the screen (5) the selected treatment appears indicated, so that when the mentioned push- button (13) is pressed Again, the treatment cycle is started, being it possible to restart in the same way all the needed circles, till in each case the treatment to be applied is completed.

The application of the treatment can be interrupted at any time by pressing one (7.1) of the set of keys (7), reflecting then the interruption on the screen (5); and to continue with the treatment from where it was interrupted, the push-button (13) should be pressed, with which the interrupted cycle is started again.
The device has a series of programming "menus", the access to which is obtained by pressing a key (8.1) of the set (8), which by means of successive pushing selects the different available "menus": TRATMENT- PASSWORD-APPLICATION- TOTALIZER-TECHNICAL SERVICE.

Once a "menu" has been selected, its activation is reached by pressing another key (8.2) of the set (8).

When the "menu" TREATMENT is selected and activated, the selected treatment appears on the screen (5) it can be changed with key (8.1), allowing to select the treatment of interest, from the ones enabled on the set, among the ones which are available (such as tobacco-addiction, stress, obesity, insomnia, migraines, impotence, pain, drug-addiction and alcoholism); appearing on the screen (5) only the treatments which have been activated.

When the wished treatment appears on the screen (5) to activate the mentioned treatment it is necessary to press push-button (8.3) from the set (8). The device shows the selected treatment momentarily on the screen (5), returning to the initialself-cheking state, while by means of some beeps it is confirmed that the selection has been carried out.

The "menu" PASSWORD has two selection possibilities, ACTIVATED or CANCELLED, so that when pressing push-button (7.2) of the set (7) the user's Password is cancelled, being then the equipment operative without the need to introduce the user's password.

On the contrary, by pressing another key (7.3) of the set (7), the password function remains activated, appearing then on the screen (5) the password request for the equipment to be operative to carry out the treatments.

The user can introduce the wished password by pressing a combination of five keys (8.3), so that in connection with the corresponding treatment a password remains pre-fixed, without which the equipment cannot be operative for the mentioned treatment.

The "menu" APPLICATION in turn has two selection possibilities, CONT or IMPULS, so that when pressing the key (7.3) the equipment carries out a continuous infrared emission for the treatment to be carried out; while pushing key (7.2) the equipment carries out an infrare emission process through impulses for the treatment.
By means of the TOTALIZING "menu" the active treatment can be seen on the screen, which is the one being carried out at the moment, or any other previously selected treatment, appearing on the screen (5) for each case a number of 6 digits, which represents the total amount of cycles of the selected testament carried out till that moment.
By means of key (7.4) of the set (7), a resetting of the equipment can be carried out to the initial waiting state forits disposition to carry out another treatment, or to disconnect the system by means of the push-button (3).

## Claims

1. Thermoestimulating device for therapeutic treatments, **characterized in that** it is made up of an equipment which includes an electronic unit (1) of digital or analogue type, in the functions of control module for the process of an infrared emission, which can be projected from an issuing element (2) for the application on the treatment areas or points, being selectively in a continuous way or through impulses for its application.

2. Thermoestimulating device for therapeutic treatments, according the first claim, **characterized in that** the electronic unit (1) has a connecting push-button (3), a screen (5) to visualize and inform on the operations and a beeper (6) for acoustic indications, including a group of keys (7) to establish the functions to develop and a group of keys (8) to program the equipment, with indicating devices (9), (10) and (11) of the activation state during the operation.

3. Thermoestimulating device for therapeutic treatments, according the first and second claims, **characterized in that** the set of keys (7) includes four different functions can be selected related to the operative activity of the equipment, with visual indication on the screen (5) in each case of the corresponding function.

4. Thermoestimulating device for therapeutic treatments, according the first and second claims, **characterized in that** the set of keys (8) includes three keys (8.1), (8.2) and (8.3), by means of which different programmes can be determined for the operation of the equipment, with visual indication on the screen (5) in each case of the corresponding programme.

5. Thermoestimulating device for therapeutic treatments, according the first and fourth claims, **characterized in that** the key set (7) and (8) an access password can be determined to activate the equipment operation in each application treatment.

6. Thermoestimulating device for therapeutic treatments, according the first claim, **characterized in that** the issuing device (2) is an independent element which can be connected to the electronic unit (1) by means of a corresponding terminal (15), having the mentioned issuing device (2) a respective push-button for its operative activation.
